# EUROPEAN PATENT APPLICATION

(11) **EP 4 756 013 A1**
(43) Date of publication of application: **10.06.2026**
(21) Application number: 24849241.5
(22) Date of filing: 01.08.2024
(51) Int. Cl.: C12N 5/0783, C12N 5/0789

(54) **METHOD FOR PRODUCING NATURAL KILLER CELLS**

(30) Priority: 02.08.2023 JP 2023126651
(71) Applicant: Healios K.K., Tokyo 100-0006 (JP)
(72) Inventor: KURACHI, Kenji, Tokyo 100-0006 (JP); YAMADA, Masashi, Tokyo 100-0006 (JP); NINOMIYA, Naoto, Tokyo 100-0006 (JP); INAMORI, Masakazu, Tokyo 100-0006 (JP); KIMURA, Hironobu, Tokyo 100-0006 (JP); TAMURA, Kouichi, Tokyo 100-0006 (JP)
(74) Representative: Weickmann & Weickmann PartmbB
(86) International application number: PCT/JP2024/027484
(87) International publication number: WO 2025/028596

(57) **Abstract**

The present invention provides a method for producing a natural killer (NK) cell or a progenitor cell thereof from a pluripotent stem cell, including
a) a step of inducing a hematopoietic progenitor cell (HPC) from a pluripotent stem cell in vitro;
b) a step of expanding HPC in vitro; and
c) a step of inducing an NK cell or a progenitor cell thereof from HPC in vitro, wherein

a histone methyltransferase and/or an aryl hydrocarbon receptor (AHR) are/is inhibited in one or more steps of steps a) to c); and
a method including
(1) a step of forming pluripotent stem cell spheres having an average particle size of not less than 200 µm in a first medium;
(2) a step of inducing the pluripotent stem cell spheres obtained in step (1) into a cell population containing HPCs by three-dimensional culture using a second medium containing a histone methyltransferase inhibitor and/or an AHR antagonist; and
(3) a step of inducing the cell population containing HPCs obtained in step (2) into a cell population containing NK cells or progenitor cells thereof by three-dimensional culture using a third medium containing a histone methyltransferase inhibitor and/or an AHR antagonist,

wherein one or more steps of steps (1) to (3) are performed by a perfusion culture method, preferably steps (1) to (3) are performed by a continuous perfusion culture method.

## Description

### [Technical Field]

The present invention relates to methods for producing natural killer cells (hereinafter sometimes to be abbreviated as "NK cells") useful in the medical field from pluripotent stem cells efficiently in large amounts.

### [Background Art]

There are many reports on methods for inducing NK cells from pluripotent stem cells. For example, Patent Literature 1 describes a method for inducing NK cells from pluripotent stem cell spheres via hematopoietic progenitor cells (hereinafter sometimes to be abbreviated as "HPCs"). The present inventors found that NK cells can be induced efficiently and rapidly by increasing the average particle size of pluripotent stem cell spheres formed in three-dimensional culture than conventional levels and further combining with a perfusion culture method, and filed a patent application (Patent Literature 2).

On the other hand, regarding the studies of medium additives, for example, Patent Literature 3 discloses a method for inducing NK cells by using a notch ligand. Patent Literature 4 discloses a method for inducing lymphoid lineage cells which uses a notch ligand and an inhibitor of histone methyltransferase EZH1 as a constituent element of polycomb complex. Furthermore, Patent Literature 5 reports a method for inducing progenitor T cells by using a notch ligand and StemRegenin 1 (SR1) which is an aryl hydrocarbon receptor (AHR) antagonist, and Patent Literature 6 reports a method for inducing NK cells from CD34-positive cells by using SR1 and thrombopoietin (TPO).

However, the NK cell induction effect of histone methyltransferase inhibition or AHR inhibition alone is not clear, and no reports exist as regards the effect of combining histone methyltransferase inhibition with AHR inhibition.

With this background, an NK cell induction method with further enhanced induction efficiency has been demanded.

### [Citation List]

### [Patent Literature]

[Patent Literature 1]
   WO 2020/086889
[Patent Literature 2]
   WO 2023/145922
[Patent Literature 3]
   US 8,518,397
[Patent Literature 4]
   WO 2018/048828
[Patent Literature 5]
   WO 2021/200901
[Patent Literature 6]
   WO 2017/046142

### [Summary of Invention]

### [Technical Problem]

It is therefore an object of the present invention to provide a method for inducing NK cells from pluripotent stem cells more efficiently than conventional techniques. A further object of the present invention is to provide a novel method for inducing NK cells from pluripotent stem cells that can further enhance the effect of improving induction efficiency achieved by a perfusion culture method, by combining with said method.

### [Solution to Problem]

The present inventors have conducted various studies in an attempt to achieve the above-mentioned objects and found that NK cells can be induced efficiently and rapidly by, in a method for inducing NK cells in vitro from pluripotent stem cells via HPCs, inhibiting histone methyltransferase and/or AHR in one or more of the steps of:
a) a step of inducing HPC from a pluripotent stem cell;
b) a step of expanding HPC; and
c) a step of inducing an NK cell or a progenitor cell thereof from HPC.

Furthermore, the present inventors have performed steps a) to c) using three-dimensional culture, set the average particle size of the pluripotent stem cell spheres formed in step a) to 200 µm or more, induced a cell population containing HPCs from the spheres, and combined perfusion culture therewith for at least a part of the subsequent culture. As a result, they have successfully induced NK cells even more efficiently and completed the present invention.

Accordingly, the present invention provides the following.

### [Item 1]

A method for producing a natural killer (NK) cell or a progenitor cell thereof from a pluripotent stem cell, comprising:
a) a step of inducing a hematopoietic progenitor cell (HPC) from a pluripotent stem cell in vitro;
b) a step of expanding HPC in vitro; and
c) a step of inducing an NK cell or a progenitor cell thereof from HPC in vitro, wherein
a histone methyltransferase and/or an aryl hydrocarbon receptor (AHR) are/is inhibited in one or more steps of steps a) to c).

### [Item 2]

The method of Item 1, wherein the histone methyltransferase catalyzes the addition of a methyl group to histone 3 lysine residue 27 (H3K27).

### [Item 3]

The method of Item 1, wherein the histone methyltransferase is EZH1 (enhancer of zeste homolog 1) and/or EZH2 (enhancer of zeste homolog 2).

### [Item 4]

The method of any one of Items 1 to 3, wherein the histone methyltransferase is inhibited by a small molecule inhibitor.

### [Item 5]

The method of Item 4, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

### [Item 6]

The method of any one of Items 1 to 5, wherein the inhibition of AHR has antagonist activity against AHR.

### [Item 7]

The method of any one of Items 1 to 6, wherein the AHR is inhibited by a small molecule inhibitor.

### [Item 8]

The method of Item 7, wherein the small molecule inhibitor is SR1 (StemRegenin 1), CH-223191, GNF-351, 7-ketocholesterol, CB7993113, 6,2',4'-trimethoxyflavone, PD98059, or BAY 2416964.

### [Item 9]

The method of any one of Items 1 to 8, wherein one or more steps of the steps a) to c) are performed by a perfusion culture method.

### [Item 10]

A method for producing an NK cell or a progenitor cell thereof from a pluripotent stem cell, comprising
(1) a step of forming pluripotent stem cell spheres having an average particle size of not less than 200 µm in a first medium;
(2) a step of inducing the pluripotent stem cell spheres obtained in step (1) into a cell population containing HPCs by three-dimensional culture using a second medium containing a histone methyltransferase inhibitor and/or an AHR antagonist; and
(3) a step of inducing the cell population containing HPCs obtained in step (2) into a cell population containing NK cells or progenitor cells thereof by three-dimensional culture using a third medium containing a histone methyltransferase inhibitor and/or an AHR antagonist,
wherein one or more steps of steps (1) to (3) are performed by a perfusion culture method.

### [Item 11]

The method of Item 10, wherein the histone methyltransferase catalyzes the addition of a methyl group to H3K27.

### [Item 12]

The method of Item 10, wherein the histone methyltransferase is EZH1 and/or EZH2.

### [Item 13]

The method of any one of Items 10 to 12, wherein the histone methyltransferase inhibitor is a small molecule inhibitor.

### [Item 14]

The method of Item 13, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

### [Item 15]

The method of any one of Items 10 to 13, wherein the AHR antagonist is a small molecule inhibitor.

### [Item 16]

The method of Item 15, wherein the small molecule inhibitor is SR1, CH-223191, GNF-351, 7-ketocholesterol, CB7993113, 6,2',4'-trimethoxyflavone, PD98059, or BAY 2416964.

### [Item 17]

The method of any one of Items 10 to 16, wherein the steps (1) to (3) are performed without using a three-dimensional culture carrier or an extracellular substrate.

### [Item 18]

The method of Item 17, wherein the perfusion culture in step (1) is performed using a separation membrane having a fine pore size of 15 - 75 µm.

### [Item 19]

The method of Item 17 or 18, wherein the perfusion culture in step (2) is performed using a separation membrane having a fine pore size of 45 - 225 µm.

### [Item 20]

The method of any one of Items 17 to 19, wherein the perfusion culture in step (3) is performed using a separation membrane having a fine pore size of 0.2 - 10 µm.

### [Item 21]

The method of any one of Items 16 to 20, wherein the steps (1) to (3) are performed by a continuous perfusion culture method.

### [Item 22]

The method of any one of Items 16 to 21, wherein the first medium comprises a ROCK inhibitor.

### [Item 23]

The method of any one of Items 16 to 22, wherein the second medium comprises VEGF, BMP4, and a GSK3β inhibitor.

### [Item 24]

The method of Item 23, wherein the second medium further comprises a ROCK inhibitor or bFGF.

### [Item 25]

The method of any one of Items 16 to 22, wherein the second medium comprises SCF, a TGFβ/Smad inhibitor, and VEGF.

### [Item 26]

The method of Item 25, wherein the second medium further comprises a ROCK inhibitor or bFGF.

### [Item 27]

The method of any one of Items 16 to 22, wherein the second medium comprises SCF and Flt3L.

### [Item 28]

The method of Item 27, wherein the second medium further comprises at least one selected from a ROCK inhibitor, IL-3, and IL-7.

### [Item 29]

The method of any one of Items 16 to 22, wherein the three-dimensional culture in step (2) comprises
(2-1) a culture step using a medium comprising VEGF, BMP4, and a GSK3β inhibitor as the second medium,
(2-2) a culture step using a medium comprising SCF, a TGFβ/Smad inhibitor, and VEGF as the second medium, and
(2-3) a culture step using a medium comprising SCF and Flt3L as the second medium.

### [Item 30]

The method of any one of Items 16 to 29, wherein the third medium comprises IL-15 and SCF.

### [Item 31]

The method of Item 30, wherein the third medium further comprises IL-7 and Flt3L.

### [Item 32]

The method of any one of Items 16 to 31, which does not require a step of sorting NK cells.

### [Advantageous Effects of Invention]

The present invention enables the efficient production of large amounts of NK cells at a time from pluripotent stem cells by inhibiting histone methyltransferase and/or AHR signal. By performing this method using three-dimensional culture in combination with perfusion culture enables, NK cells can be produced extremely efficiently and rapidly. The NK cells produced by said method exhibit high cytotoxic activity against various cancer cells, and the activity is maintained without decrease even when frozen, thus enabling large-scale clinical application.

### [Brief Description of Drawings]

[Fig. 1]

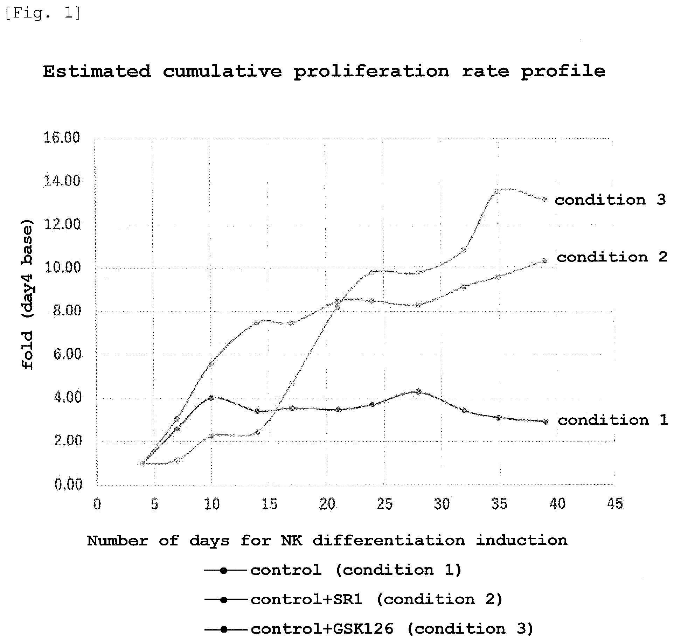

   Fig. 1 is a diagram showing daily changes in viable NK cell density when the hematopoietic progenitor cells obtained in step (2) were floating cultured under various conditions and induced to differentiate into NK cells.
[Fig. 2]

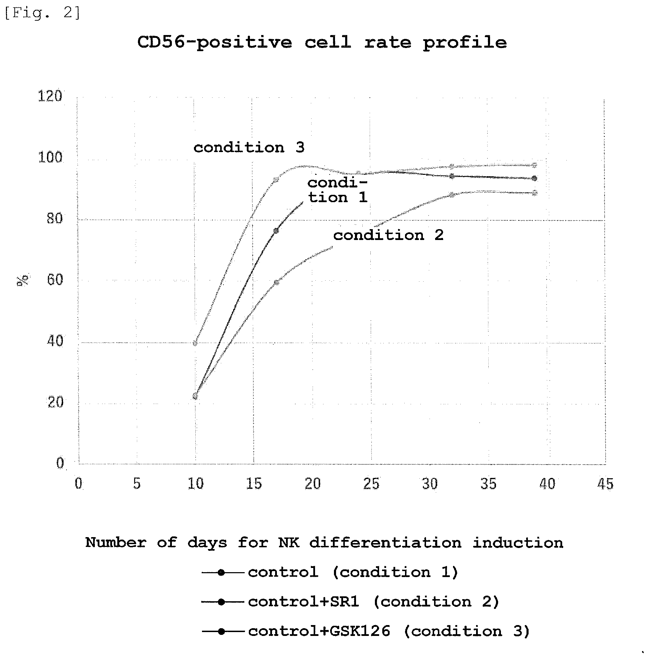

   Fig. 2 is a diagram showing daily changes in CD56-positive cells when the hematopoietic progenitor cells obtained in step (2) were floating cultured under various conditions and induced to differentiate into NK cells.
[Fig. 3]

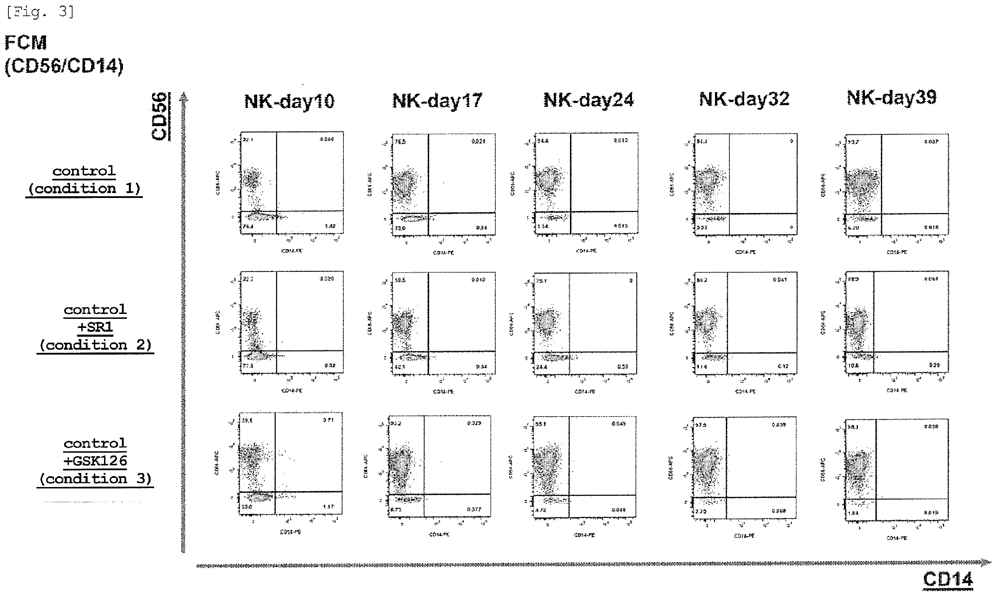

   Fig. 3 shows FACS analysis results showing CD56 and CD14 expression in cell populations 10, 17, 24, 32, and 39 days after differentiation induction, when the hematopoietic progenitor cells obtained in step (2) were floating cultured under various conditions and induced to differentiate into NK cells.

### [Description of Embodiments]

The present invention provides a method for producing an NK cell or a progenitor cell thereof from a pluripotent stem cell, including
a) a step of inducing HPC from a pluripotent stem cell in vitro;
b) a step of expanding HPC in vitro; and
c) a step of inducing an NK cell or a progenitor cell thereof from HPC in vitro, wherein
histone methyltransferase and/or AHR are/is inhibited in one or more steps of steps a) to c) (hereinafter sometimes to be referred to as "the method of the present invention").

The pluripotent stem cells used in the method of the present invention include, but are not limited to, embryonic stem (ES) cells and induced pluripotent stem (iPS) cells. For example, when using iPS cells, the method of producing iPS cells and the cells from which they are derived are not particularly limited. The method of culturing iPS cells is also not particularly limited, and may be two-dimensional culture or three-dimensional culture. Furthermore, cryopreserved iPS cells may also be used.

The method of the present invention induces an NK cell or a progenitor cell thereof in vitro from a pluripotent stem cell via HPC. That is, the method of the present invention includes
a) a step of inducing HPC from a pluripotent stem cell in vitro;
b) a step of expanding HPC in vitro; and
c) a step of inducing an NK cell or a progenitor cell thereof from HPC in vitro.
Here, in step a) and step b), induction of differentiation into HPC to maintenance and expansion of HPC may be performed continuously using the same or a series of media. In this case, the steps, including the maintenance and expansion phase, can be considered a process of inducing HPC from a pluripotent stem cell. Alternatively, the HPC obtained in step a) may be temporarily stocked, for example by cryopreservation, and then stored for a certain period of time, after which the HPC stock may be subjected to step b). For example, when step a) and step b) are performed using three-dimensional culture by continuous perfusion culture (e.g., in the preferred embodiment of the present invention described below), the process of inducing a cell population containing HPCs from pluripotent stem cell spheres and further purifying and expanding the HPCs can be performed as a single step using the same or a series of media (step (2) in the preferred embodiment of the present invention).

Pluripotent stem cells can be induced to differentiate into NK cells via HPCs by a method known per se, for example, the methods described in WO 2020/086889, Biochem Biophys Res Commun. 515(1): 1-8 (2019), Methods Mol Biol. 2048: 107-119 (2019), and the like.

For example, step a) can be performed by culturing pluripotent stem cells in a medium capable of inducing pluripotent stem cells into HPCs. Examples of such medium include, but are not limited to, a medium containing vascular endothelial growth factor (VEGF), bone morphogenetic protein 4 (BMP4), and a glycogen synthase 3β (GSK3β) inhibitor, a medium containing stem cell factor (SCF) and a transforming growth factor β (TGFβ)/Smad inhibitor, and a medium containing SCF and Flt3 ligand (Flt3L).

For example, step b) can be performed by culturing HPCs in a medium capable of maintaining and expanding HPCs. Examples of such medium include, but are not limited to, media containing SCF and Flt3 ligand (Flt3L), and the like.

For example, step c) can be performed by culturing HPCs in a medium that can induce HPCs into NK cells. Examples of such medium include, but are not limited to, media containing IL-15 and SCF, and the like.

Examples of the basal medium used in the preceding steps a) to c) include DMEM/F-12, HEPES (Thermo Fisher Scientific), Essential 6 medium (Thermo Fisher Scientific), Stem Pro-34 SFM (Thermo Fisher Scientific), AIM-V Medium (Thermo Fisher Scientific), Stemline^{®} II (Sigma-Aldrich), and ALyS505N-0 (Cell Science & Technology Institute, Inc.). Those skilled in the art can appropriately select a preferred basal medium for each step.

The culture period in steps a) and b) is generally 5 to 20 days, preferably about 10 to 18 days.

The method of the present invention is characterized by inhibiting histone methyltransferase and/or AHR in one or more of the preceding steps a) to c).

The histone methyltransferase targeted in the method of the present invention is not particularly limited, but is preferably one that catalyzes the addition of a methyl group to histone 3 lysine residue 27 (H3K27), more preferably EZH1 (enhancer of zeste homolog 1) and/or EZH2 (enhancer of zeste homolog 2).

Histone methyltransferase can be inhibited by adding a substance having inhibitory activity against the enzyme to a medium. The "inhibition of histone methyltransferase" here may be performed by any method as long as the methyl group transfer reaction to histone, which is catalyzed by the enzyme, is inhibited eventually, and may involve inhibition of not only the function but also expression of the enzyme. Examples of the substance that inhibits the function of histone methyltransferase include, but are not limited to, small molecule inhibitors, antibodies, and aptamers, and examples of the substance that inhibits the expression of histone methyltransferase include, but are not limited to, nucleic acids complementary to the enzyme gene or a transcript thereof (e.g., siRNA, antisense nucleic acid, miRNA, etc.).

In one preferred embodiment, the histone methyltransferase inhibitor is a small molecule inhibitor. As the small molecule inhibitor, an inhibitor known per se is appropriately selected depending on the target histone methyltransferase. Examples of the inhibitors of EZH1 and/or EZH2 include EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, GSK126, and the like. Alternatively, examples of inhibitors that are expected to inhibit PRC1.1 to suppress the recruitment of PRC2, thus inhibiting methylation of histone include FT671, XL177A, P5091, HBX19818, Parthenolide, GNE-6640, XL188, L55, and the like.

Among these, preferred are GSK503, EPZ005687, EI1, Tazemetostat (EPZ-6438), GSK126, and the like, and more preferred is GSK126.

The concentration of the histone methyltransferase inhibitor to be added is not particularly limited as long as it can efficiently and rapidly induce NK cells and does not adversely affect the cells, and a preferred concentration can be appropriately selected depending on the kind of the inhibitor. For example, the concentration can be selected from the range of about 10 nM to about 5 µM, preferably about 100 nM to about 2 µM, more preferably about 500 nM to about 1 µM. Any one kind of the histone methyltransferase inhibitor may be used alone, or two or more kinds thereof may be used in combination.

While not wishing to be bound by any particular theory, it is suggested that the use of an inhibitor of histone methyltransferase such as EZH1 and/or EZH2 (e.g., GSK126) accelerates the onset of NK cell differentiation when inducing differentiation from HPC into NK cell, and that the longer the NK cell differentiation induction time, the higher the cell proliferation efficiency.

The aryl hydrocarbon receptor (AHR) is a transcription regulatory factor that, upon binding to an aromatic hydrocarbon compound as its ligand, forms a complex that is translocated into the nucleus and regulates the expression of target genes. AHR can be inhibited by adding, to the medium, a substance having an activity to inhibit the transcription factor. The "inhibition of AHR" here may be performed by any method as long as the transcription factor eventually inhibits transcriptional regulation of the target gene whose expression is regulated by the transcription factor, and may involve inhibition of not only the function but also expression of the transcription factor. Examples of the substance that inhibits the function of the transcription factor include, but are not limited to, small molecule inhibitors, antibodies, aptamers, and decoy nucleic acids, and examples of the substance that inhibits the expression of AHR include, but are not limited to, nucleic acids complementary to the AHR gene or a transcript thereof (e.g., siRNA, antisense nucleic acid, miRNA, etc.).

In one preferred embodiment, the AHR inhibitor (antagonist) is a small molecule inhibitor. The small molecule inhibitor may be appropriately selected from known inhibitors. In a preferred embodiment, examples include SR1 (StemRegenin 1), CH-223191, GNF-351, 7-ketocholesterol, CB7993113, 6,2' ,4'-trimethoxyflavone, PD98059, and BAY 2416964. SR1, CH-223191, GNF-351, CB7993113, 6,2',4'-trimethoxyflavone and the like are preferred, with SR1 being more preferred.

The concentration of the AHR inhibitor to be added is not particularly limited as long as it can efficiently and rapidly induce NK cells and does not adversely affect the cells, and a preferred concentration can be appropriately selected depending on the kind of the inhibitor. For example, the concentration can be selected from the range of about 100 nM to about 10 µM, preferably about 500 nM to about 5 µM, more preferably about 1 µM to about 5 µM. Any one kind of the AHR inhibitor may be used alone, or two or more kinds thereof may be used in combination.

While not wishing to be bound by any particular theory, it is suggested that the use of an inhibitor of AHR (e.g., SR1) can further increase cell proliferation efficiency in the early stages of inducing differentiation of HPC into NK cell.

As such, it is suggested that histone methyltransferase inhibition and AHR inhibition have different points of action to efficiently induce NK cells, and therefore, a synergistic effect can be expected by inhibiting both. Therefore, in the method of the present invention, a histone methyltransferase inhibitor and an AHR inhibitor may be used alone, and it is also preferable to use both in combination. When a histone methyltransferase inhibitor and an AHR inhibitor are used in combination, any of combinations of the above-mentioned histone methyltransferase inhibitors and AHR inhibitors may be used, and combined use of GSK126 and SR1 is particularly preferred.

The inhibition of histone methyltransferase and/or AHR is not particularly limited as long as it is performed in at least one of steps a) to c). In one embodiment, it can be performed in all of steps a) to c). For example, as a suitable timing for performing the inhibition, from the latter half of step a) to the first half of step c) may further increase the induction efficiency of NK cells.

The method of the present invention is intended for the efficient production of large amounts of NK cells useful in the medical field from pluripotent stem cells. Thus, while this method can also be performed using two-dimensional culture, it is preferable to perform the method by three-dimensional culture, since industrial scale-up is facilitated thereby. Manual medium exchange poses a risk of affecting the quality of the final product due to the artificial operation, as well as an increase in the production costs due to the increased number of steps is also expected. Therefore, the method of the present invention is preferably performed using a perfusion culture method, which does not require manual medium exchange.

Therefore, in a preferred embodiment, the method of the present invention is a method for producing an NK cell or a progenitor cell thereof from a pluripotent stem cell, including
(1) a step of forming pluripotent stem cell spheres having an average particle size of not less than 200 µm in a first medium;
(2) a step of inducing the pluripotent stem cell spheres obtained in step (1) into a cell population containing HPCs by three-dimensional culture using a second medium containing a histone methyltransferase inhibitor and/or an AHR antagonist; and
(3) a step of inducing the cell population containing HPCs obtained in step (2) into a cell population containing NK cells or progenitor cells thereof by three-dimensional culture using a third medium containing a histone methyltransferase inhibitor and/or an AHR antagonist,
wherein one or more steps of steps (1) to (3) are performed by a perfusion culture method.

In a particularly preferred embodiment, the aforementioned steps (1) to (3) are performed by continuous perfusion culture. In this embodiment, step (2) and step (3) are performed continuously in a single culture system by continuous perfusion culture, without including, between step (2) and step (3), a step of temporarily storing the cell population containing HPCs or HPCs purified from the cell population by, for example, cryopreservation or the like. Therefore, in step (2), the differentiation induction of HPCs from pluripotent stem cells (the aforementioned step a)) and the expansion of HPCs (the aforementioned step b)) are performed continuously as a single step.

In a preferred embodiment, moreover, pluripotent stem cells are cultured in three-dimensional culture, and therefore, the aforementioned step (1) is performed prior to step (2) (the aforementioned step a)) as a pre-step in which pluripotent stem cells are three-dimensionally cultured to produce pluripotent stem cell spheres.

The three-dimensional culture method and perfusion culture method are described in detail below.

### (Three-dimensional culture method)

The three-dimensional culture method in the present invention refers to a method in which cells are first formed into spheres (spheroids) and then cultured by making them suspended in a medium (sometimes to be referred to as "the three-dimensional floating culture method" in the present specification).

The three-dimensional floating culture method is one of the cell culture methods and characterized by culturing floating cells, spheres (spheroids), or carriers for three-dimensional culture with cells attached thereto, while developing them three-dimensionally using a stirring blade or shaker. Therefore, the three-dimensional floating culture can maximize the number of cells per space as compared to two-dimensional culture, in which cells are only cultured on the bottom surface of the culture container. Furthermore, it can make the environment of the culture medium uniform by mixing three-dimensionally using a stirring blade or shaker. In the three-dimensional culture, efficient culture in a uniform environment is possible by combining with various detectors such as pH sensor, dissolved oxygen sensor, and the like.

The facts that the culture space can be utilized to the maximum extent and that culture can be performed in a uniform environment are also useful for scaling up of culture assuming commercial production. In addition, when culturing floating cells or spheroids, this culture method can be an advantageous culture method in terms of cost because it does not require specially processed plastic carriers for cell culture or an extracellular matrix required for many cultured cells. Furthermore, it is also one of the characteristics that culture can be performed in an environment closer to that of the living body than two-dimensional culture, in which cells are cultured by adhering them to a flat surface. Thus, it is a useful means for producing various cell products derived from pluripotent stem cells (e.g., ES cells, iPS cells, and the like) that require differentiation induction into various cells.

### (Perfusion culture method)

The three-dimensional culture method of the present invention can more effectively induce NK cells when combined with a perfusion culture method.

The perfusion culture method is one of the continuous culture methods, and is a method capable of achieving continuous supply of new nutrients and removal of waste products, which are the purposes of medium exchange, by simultaneously supplying a given amount of new medium to the culture medium in the culture container and removing a given amount of the medium.

The use of perfusion culture method eliminates the need for medium exchange, which is expected to drastically reduce the workload. In addition, because this method is generally performed mechanically, it does not cause environmental changes such as temperature change, pH change, dissolved oxygen concentration change, and dissolved carbon dioxide concentration change that occur with manual medium exchange. This enables a longer-term culture in the same container, and as a result, the cell density that can be maintained in the same container can be maintained at a high level. In addition, the perfusion culture method can also maintain a constant concentration of various growth factors, and the like secreted by cells, which are removed by general medium exchange, and is also a culture method that enables culture in an in vivo-like environment that mimics the blood circulation system.

The separation membrane used in perfusion culture desirably has a certain fine pore size and properties (e.g., hydrophobicity, etc.) that prevent the target cell population from escaping to the outside of the culture system. The fine pore size of the separation membrane can be appropriately selected according to the target cell population, but since various differentiated cells are induced inside and outside the sphere in the NK cell induction step, the selection of the fine pore size generally requires various considerations, and optimization thereof is very difficult.

While specific fine pore sizes for each step are described later, an appropriate fine pore size for the perfusion culture of the present invention is a fine pore size of 15 - 75 µm for step (1), a fine pore size of 45 - 225 µm for step (2), and a fine pore size of 0.2 - 10 µm for step (3).

While the material of the separation membrane is not particularly limited, and a material that does not affect the cells or the components in the culture medium is preferably used. Examples of such material include metal materials such as SUS304, SUS316, and the like, natural fibers such as cellulose fiber and the like, and chemical fibers such as polysulfone fiber, polyethersulfone fiber, and the like.

The three-dimensional culture method of the present invention is characterized in that a cell population, such as cell spheres or cells, present in a culture system is perfusion cultured in each step described below. The conditions for each perfusion culture are as described below for each step. The perfusion culture in the present invention permits "continuous perfusion culture".

The "continuous perfusion culture" here refers to the process of simply replacing the membrane between each step, without a washing step generally performed when exchanging the medium, and supplying the medium for the next step while removing the medium from the previous step.

For example, in step (1), the medium is continuously exchanged by supplying the first medium described below while simultaneously withdrawing the medium, and when switching from step (1) to step (2), the medium is continuously exchanged from the first medium to the second medium by supplying the second medium described below while simultaneously withdrawing the first medium. In step (2), the medium is continuously exchanged by supplying the second medium while simultaneously withdrawing the medium, and when switching from step (2) to step (3), the medium is continuously exchanged from the second medium to the third medium by supplying the third medium described below while simultaneously withdrawing the second medium.

### (Step of forming pluripotent stem cell spheres: step (1))

Step (1) in a preferred method of the present invention is a step of forming pluripotent stem cell spheres having an average particle size of not less than 200 µm in the first medium.

The pluripotent stem cells used in the present invention for forming pluripotent stem cell spheres include, but are not limited to, ES cells and iPS cells, as described above.

The sphere formation is performed under floating culture conditions. The floating culture conditions are not particularly limited as long as the environment of the culture medium can be made uniform by three-dimensional mixing using a stirring blade or shaker. The container used for floating culture is not particularly limited, and a container with a stirring mechanism such as a stirring blade agitator or a stirring blade up-and-down agitator is used. The seeding density of the pluripotent stem cells in the sphere formation of the pluripotent stem cells of the present invention is within the range of 1.0×10⁴ cells/mL to 1.0×10⁶ cells/mL. When the density is higher than this, the shape of the sphere may become too large, which may affect the subsequent induction efficiency. Among them, the cells are more preferably seeded within the range of 5.0×10⁴ cells/mL to 2.0×10⁵ cells/mL.

The culture medium used for the formation of pluripotent stem cell spheres in this step (1) is not particularly limited as long as it is a culture medium used for the maintenance culture of pluripotent stem cells. Examples of such culture medium include media capable of feeder-free culture, such as StemFit (registered trademark) AK03N (Ajinomoto Healthy Supply Co., Ltd.), mTeSR^{™}1 (STEMCELL Technologies Inc.), and the like. Among them, the culture medium used in the preferred method of the present invention is a medium capable of feeder-free culture and containing a ROCK inhibitor. In particular, when iPS cell is used as the pluripotent stem cell, it is preferable to use a medium such as StemFit (registered trademark) AK03N (Ajinomoto Healthy Supply Co., Ltd.), and the like.

Examples of the ROCK inhibitor in the present invention include Y27632, thiazovivin, and the like. In iPS cell culture, Y27632 is generally used as a ROCK inhibitor, and in the present invention, the concentration thereof is preferably 1 to 20 µM, and it is preferable to maintain the added state for 2 days after cell seeding.

The culture period required for sphere formation is not limited as long as spheres are formed. The spheres in the present invention are not of the type formed by forcibly forming spheres by inserting and associating pluripotent stem cells into dimples or the like, but are formed as the cells proliferate, and therefore require a certain period of time. For example, 2 to 10 days from cell seeding are preferred, and 4 to 7 days are more preferred.

The above-mentioned formation of the spheres can be adjusted by the stirring conditions, seeding density conditions, and culture period. The average particle size of the spheres in step (1) of the method of the present invention is generally adjusted to not less than 200 µm, specifically within the range of 200 to 600 µm, but is preferably adjusted to 200 to 500 µm to further improve the production efficiency of NK cells, and more preferably adjusted to 200 to 400 µm.

When the average particle size is less than 200 µm, the number of culture days becomes insufficient and a sufficient number of cells are not obtained, which is undesirable because step (2) is affected. Furthermore, when the average particle size is too large, the supply of sufficient nutrient sources and oxygen to the center of the sphere is blocked, which is undesirable because it induces cell death such as necrosis (e.g., Cells Tissues Organs 196.1 (2012): 34-47).

The sphere formation step (1) is performed by a perfusion culture method. In step (1), the perfusion culture may be performed from the time of seeding the pluripotent stem cells, but it is preferable to perform the perfusion culture about one day after the initial seeding of the pluripotent stem cells, and more preferably, two days after the initial seeding. This is because spheroids are formed after one or two days of culture, and under these conditions the average particle size is not less than 200 µm. When the perfusion culture is performed before the formation of spheroids, the spheroids may not be formed satisfactorily.

In addition, in this step (1), the fine pore size of the separation membrane in the perfusion culture is preferably 15 to 75 µm, and more preferably 25 to 45 µm. This is because the use of a separation membrane with a pore size not exceeding the desired spheroid size makes it possible to perform the perfusion culture while keeping a certain number of spheroids in the culture container. Therefore, preferably, step (1) includes:
(1-1) a step of forming pluripotent stem cell spheres having an average particle size of 200 µm or more in a first medium; and
(1-2) a step of maintenance culturing the pluripotent stem cell spheres having an average particle size of not less than 200 µm obtained in step (1-1) by a perfusion culture method. That is, the fine pore size of the separation membrane in the perfusion culture in step (1) (or step (1-2)) is preferably 15 to 75 µm, more preferably 25 to 45 µm.

The medium exchange in perfusion culture is performed at a constant dilution rate. The sphere formation process of pluripotent stem cells is preferably performed at a dilution rate of 0.01 to 0.2 hr⁻¹, which can be adjusted with reference to the cell seeding density, the glucose concentration, the lactate concentration, the glutamine concentration and the glutamic acid concentration of the culture supernatant, and the like. The dilution rate can be adjusted by placing the culture container, the supply bottle, and the discharge bottle on a balance or a load cell, or by controlling the rotation speed of the peristaltic pump.

In this step (1), the temperature, pH, and dissolved oxygen concentration can be controlled to any desired value for sphere formation of pluripotent stem cells by using a temperature sensor, a pH sensor, a dissolved oxygen sensor, and the like.

The culture temperature is preferably controlled between 35 and 39°C, more preferably 36 and 38°C. The pH is controlled by the inflow of aseptically treated compressed air, or aseptically treated carbon dioxide gas, or a pH adjuster, or the dilution rate of the perfusion culture medium, and the control value is preferably controlled between 6.8 and 8.0, more preferably 7.0 and 7.4. The dissolved oxygen concentration is controlled by aseptically treated compressed air, or aseptically treated nitrogen gas, or aseptically treated oxygen gas, and the value thereof is controlled between 0 and 6.86 mg/L, and preferably maintained at a concentration of not less than 2.00 mg/L. The gas used to maintain the pH and dissolved oxygen can be blown into the air layer above the culture medium, blown into the culture medium, or exchanged through a gas-permeable membrane, and there is no restriction.

The thus-obtained pluripotent stem cell spheres are subjected to the next step (2) to induce hematopoietic progenitor cells.

### (Step of inducing pluripotent stem cell spheres into cell population including HPCs: step (2))

Step (2) in the preferred method of the present invention is a step of inducing the spheres formed in step (1) into a cell population including HPCs by three-dimensional culture using a second medium containing a histone methyltransferase inhibitor and/or an AHR antagonist. The histone methyltransferase inhibitor contained in the second medium is as described above. In addition, the AHR antagonist contained in the second medium is as described above.

Here, the "cell population including HPCs" induced by this step refers to a cell population obtained by step (2), and is a concept that includes an HPC population induced inside or outside the sphere.

This cell population includes HPCs formed within the sphere, HPCs that have been induced and then escaped from the sphere, and cells inside or outside the sphere that are in the induction process into HPC.

Step (2) can also be performed by perfusion culture, as in step (1). The fine pore size of the separation membrane used in this step is preferably 45 to 225 µm, more preferably 45 to 100 µm. The perfusion culture in this step is performed from immediately after the start of step (2) until the end of step (2). During this time, the "pluripotent stem cell spheres" are induced toward the "cell population including HPCs".

By using a separation membrane with the above-mentioned fine pore size during step (2), perfusion culture can be performed while maintaining a cell population including certain HPC in the culture container.

The medium exchange performed along with the perfusion culture in step (2) is also performed at a certain dilution rate, similar to that described in step (1). Specifically, it is preferably performed at a dilution rate of 0.01 to 0.2 hr⁻¹, similar to step (1), and can be adjusted with reference to the cell seeding density, glucose concentration, lactate concentration, glutamine concentration and glutamic acid concentration of the culture supernatant, and the like. This dilution rate is also applied when the medium used in step (1) is exchanged with the medium used in step (2).

The second medium in step (2) is not particularly limited as long as it is a medium containing a histone methyltransferase inhibitor and/or an AHR antagonist and capable of inducing pluripotent stem cells into HPCs.

Examples of such medium include a medium containing vascular endothelial growth factor (VEGF), bone morphogenetic protein 4 (BMP4), and a glycogen synthase 3β (GSK3β) inhibitor (hereinafter referred to as medium (2-1)), a medium containing stem cell factor (SCF) and a transforming growth factor β (TGFR) /Smad inhibitor (hereinafter referred to as medium (2-2)), and a medium containing SCF and Flt3 ligand (Flt3L) (hereinafter referred to as medium (2-3)). It is also possible to design an appropriate induction medium by appropriately changing the combination and amount of each of the medium components as necessary. In particular, the timing of administration of the histone methyltransferase inhibitor and/or AHR antagonist (specifically, which one of the media (2-1) to (2-3) it is included in) can be designed appropriately. As examples of such medium, each of the media (2-1) to (2-3) is described below.

The medium (2-1) is a medium containing VEGF, BMP4, and a GSK3β inhibitor.

Here, examples of the GSK3β inhibitor include CHIR99021 and SB216763, and CHIR99021 is preferred.

The concentration of VEGF is preferably 1 to 100 ng/mL, more preferably 50 to 100 ng/mL. The concentration of BMP4 is preferably 1 to 100 ng/mL, more preferably 50 to 100 ng/mL. When CHIR99021 is used, the concentration of the GSK3β inhibitor is preferably 1 to 10 µM, more preferably 1 to 5 µM.

Furthermore, the medium (2-1) may contain a ROCK inhibitor or bFGF. When a ROCK inhibitor is added, examples of the ROCK inhibitor include those described in step (1), and Y27632 is preferred. When Y27632 is used, the concentration of the ROCK inhibitor is preferably 1 to 20 µM, more preferably 1 to 10 µM. When bFGF is added, the concentration is preferably 1 to 100 ng/mL, more preferably 10 to 50 ng/mL.

The basal medium of the medium (2-1) is not particularly limited. For example, media such as DMEM/F-12, HEPES (Thermo Fisher Scientific), and Essential 6 medium (Thermo Fisher Scientific) are preferably used.

The medium (2-2) is a medium containing SCF, a TGFβ/Smad inhibitor, and VEGF.

Here, examples of the TGFβ/Smad inhibitor include SB431542, LY2157299, and LY2109761, and SB431542 is preferred. The concentration of SCF is preferably 1 to 100 ng/mL, more preferably 20 to 50 ng/mL. When SB431542 is used, the concentration of the TGFβ/Smad inhibitor is preferably 1 to 10 µM, more preferably 1 to 5 µM. The concentration of VEGF is preferably 1 to 100 ng/mL, more preferably 50 to 100 ng/mL, similar to the medium (2-1).

Furthermore, the medium (2-2) may contain a ROCK inhibitor or bFGF. Specific examples and concentrations of each when in use are the same as those described for the medium (2-1).

The basal medium for the medium (2-2) is not particularly limited, and the same basal medium as described for the medium (2-1) can be used.

The medium (2-3) is a medium containing SCF and Flt3L. The concentration of SCF is preferably 1 to 100 ng/mL, more preferably 20 to 50 ng/mL. The concentration of Flt3L is preferably 1 to 100 ng/mL, more preferably 20 to 50 ng/mL.

Furthermore, the medium (2-3) may contain at least one selected from a ROCK inhibitor, interleukin (IL)-3, and IL-7. Examples of the ROCK inhibitor include those mentioned above, and Y27632 is preferred. When Y27632 is used, the concentration of the ROCK inhibitor is preferably 1 to 20 µM. When IL-3 is added, the concentration is preferably 1 to 100 ng/mL. When IL-7 is added, the concentration is preferably 1 to 100 ng/mL.

The basal medium for the medium (2-3) is not particularly limited, and a medium suitable for inducing HPC is used. For example, a medium containing Stem Pro-34 SFM (Thermo Fisher Scientific) to which L-glutamine or L-alanyl-L-glutamine has been added to a final concentration of 1 to 10 mM is preferably used.

While the above-mentioned media (2-1), (2-2) and (2-3) can be used alone, it is possible to more efficiently induce hematopoietic progenitor cells by exchanging the medium (2-1) or (2-2) with the medium (2-3) at an appropriate timing during step (2). For example, HPCs can be efficiently induced by culturing in the medium (2-1) or (2-2) in the first part of step (2) and in the medium (2-3) in the second part.

In this case, the times for the first part and the second part of step (2) can be appropriately set, and HPCs can be efficiently induced by setting, for example, the first part to 2 to 6 days and the second part to 3 to 14 days. The exchange of medium (2-1) or (2-2) with medium (2-3) can be performed by continuous perfusion operation or by replacing the entire amount of the medium with a new medium, and it is more desirable to perform medium exchange by continuous perfusion operation.

Furthermore, HPCs can be efficiently induced by exchanging the culture media (2-1) and (2-2) used in the first part of step (2) in this order. Specifically, culture is first performed in medium (2-1), and then in medium (2-2).

In this case, the culture time in each medium can be set appropriately. For example, HPCs can be efficiently induced by setting the culture time in medium (2-1) to 1 to 3 days and the culture time in medium (2-2) to 1 to 3 days. This exchange between medium (2-1) and medium (2-2) can be performed by continuous perfusion operation, or by replacing the entire amount of the medium with a new medium, and it is more desirable to perform medium exchange by continuous perfusion operation.

Therefore, the three-dimensional culture in step (2) is preferably a three-dimensional culture including, for example,
(2-1) a culture step using medium (2-1) as the second medium,
(2-2) a culture step using medium (2-2) as the second medium, and
(2-3) a culture step using medium (2-3) as the second medium. Respective steps (2-1) to (2-3) are preferably performed in this order.

In this step (2), as in step (1), the temperature, pH, and dissolved oxygen concentration can be controlled to any desired value for induction into hematopoietic progenitor cells by using a temperature sensor, a pH sensor, a dissolved oxygen sensor, and the like.

The histone methyltransferase inhibitor and/or AHR antagonist may be added to the medium in step (2) throughout step (2) or only for a part of the step. When the inhibitor (antagonist) is added only for a part of step (2), it can be added, for example, only in the latter half of step (2). For example, when step (2) is performed using media (2-1), (2-2), and (2-3) sequentially as the second medium, the inhibitor (antagonist) can be added to the medium only in the culture step using media (2-2) and (2-3), or only in the culture step using medium (2-3). However, addition only in the latter half of step (2) is not limited thereto. However, from the viewpoint of efficient and rapid induction of NK cells, it is preferable to add both the histone methyltransferase inhibitor and the AHR antagonist to the medium only in the culture step using medium (2-3).

When a histone methyltransferase inhibitor and/or an AHR antagonist are/is added to each medium in step (2), no particular limitation is imposed on the selection of inhibitor (antagonist) as long as it can efficiently and rapidly induce NK cells and does not adversely affect the cells. In addition, a suitable concentration can be selected depending on the kind of inhibitor. The concentration of the histone methyltransferase inhibitor can be selected from the range of generally about 10 nM to about 5 µM, preferably about 100 nM to about 2 µM, more preferably about 500 nM to about 1 µM. The concentration of the AHR antagonist can be selected from the range of generally about 100 nM to about 10 µM, preferably about 500 nM to about 5 µM, more preferably about 1 µM to about 5 µM. Any one kind of the histone methyltransferase inhibitor may be used alone, or two or more kinds thereof may be used in combination. Any one kind of the AHR inhibitor may be used alone, or two or more kinds thereof may be used in combination.

In the method of the present invention, NK cell populations can be obtained without any particular purification step from step (1) to step (3). Therefore, the cell populations obtained in step (2) may be directly subjected to the next step (3) as they are, or only HPCs may be sorted and then subjected to step (3).

The inventors have confirmed that the desired NK cells can be obtained in step (3) by using either method. Therefore, the method of the present invention may include a step of removing cells other than HPC from the "cell population including HPCs" obtained in step (2), but it is desirable to subject the "cell population including HPCs" obtained in step (2) directly to step (3).

NK cells are induced by subjecting the thus-obtained "cell population including HPCs" to step (3).

### (Step of inducing HPC into NK cell: step (3))

Step (3) in the method of the present invention is a step of inducing the "cell population including HPCs" obtained in step (2) into a "cell population including NK cells" by three-dimensional culture using a third medium containing a histone methyltransferase inhibitor and/or an AHR antagonist. The histone methyltransferase inhibitor contained in the third medium is as described above. The AHR antagonist contained in the third medium is as described above.

Here, the "cell population including NK cells" induced by this step refers to a cell population containing NK cells obtained by step (3) and cells in the induction process into NK cells.

NK cells generally exist as single cells without forming spheres. Therefore, by step (3), the single NK cells proliferates to become a cell population of many single NK cells. This cell population contains the majority of NK cells and some cells in the induction process into NK cells.

Step (3) can also be performed by perfusion culture, as in steps (1) and (2). The fine pore size of the separation membrane to be used is preferably 0.1 to 10 µm, more preferably 0.2 to 5 µm. The perfusion culture in this step is performed continuously from immediately after the start of step (3) until the end of step (3). During this time, NK cells induced from the HPC spheres are generated, and the NK cells do not remain within the spheres but are individually filtered out. Therefore, by using a separation membrane that does not exceed the cell size of the NK cells during step (3), perfusion culture can be performed while keeping a certain number of NK cells in the culture container.

Furthermore, the medium exchange in this step (3) is also performed at a constant dilution rate as described in step (1). Specifically, it is preferably performed at a dilution rate of 0.01 to 0.2 hr⁻¹, similar to step (1), and can be adjusted with reference to the cell seeding density, glucose concentration, lactate concentration, glutamine concentration and glutamic acid concentration of the culture supernatant, and the like. This dilution rate is also applied when the medium used in step (2) is exchanged with the medium used in step (3).

The third medium in step (3) is not particularly limited as long as it is a medium containing a histone methyltransferase inhibitor and/or an AHR antagonist and capable of inducing hematopoietic progenitor cells into NK cells. Examples of such a medium include a medium containing IL-15 and SCF. The concentration of IL-15 is preferably 1 to 100 ng/mL, more preferably 20 to 50 ng/mL. The concentration of SCF is preferably 1 to 100 ng/mL, more preferably 20 to 50 ng/mL. This medium may further contain one or more components selected from IL-7, Flt3L, a ROCK inhibitor, a GSK3β inhibitor, and a TGFβ receptor (TGFβR) inhibitor. Examples of the ROCK inhibitor include Y27632 and thiazovivin, and Y27632 is preferred. Examples of the GSK3β inhibitor include CHIR99021 and SB216763, and CHIR99021 is preferred. Examples of the TGFβR inhibitor include LY2157299, SB431542, and LY2109761, and LY2157299 is preferred. In a preferred embodiment, the third medium further contains IL-7 and Flt3L in addition to IL-15 and SCF.

When IL-7 is added, the concentration is preferably 1 to 100 ng/mL, more preferably 20 to 50 ng/mL. When Flt3L is added, the concentration is preferably 1 to 100 ng/mL, more preferably 20 to 50 ng/mL. When a ROCK inhibitor is added, the concentration is preferably 1 to 20 µM, more preferably 1 to 10 µM. When a GSK3β inhibitor is added, the concentration is preferably 1 to 10 nM, more preferably 1 to 5 µM. When a TGFβR inhibitor is added, the concentration is preferably 0.1 to 100 µM, more preferably 0.1 to 5 µM.

The culture period in step (3) is generally 20 to 60 days, preferably about 25 to 40 days.

In addition, when IL-7, Flt3L, a ROCK inhibitor, a GSK3β inhibitor, or a TGFβR inhibitor is added in step (3), the induction efficiency can be improved by adjusting the timing of adding these factors. For example, IL-7 and Flt3L are preferably removed on or after the 10th day of culture in step (3), more preferably on or after the 20th day. In addition, a ROCK inhibitor, a GSK3β inhibitor, and a TGFβR inhibitor are preferably added for 4 to 7 days before the end of culture.

The basal medium to be used in step (3) is not particularly limited. For example, media such as AIM-V Medium (Thermo Fisher Scientific), Stemline (registered trademark) II (Sigma-Aldrich), ALyS505N-0 (Cell Science Institute, Inc.), and Stem Pro-34 SFM (Thermo Fisher Scientific) are used, and AIM-V Medium is preferably used. Furthermore, the medium may contain human serum, fetal bovine serum (FBS), or a serum replacement. The concentration thereof is preferably 1 to 20%, more preferably 1 to 10%.

In step (3), it is preferable to further perform a step of removing the spheres. The timing of removal may be, for example, before the start of this step, during this step, or at the end of this step, but it is not particularly limited and can be performed at any time.

The spheres are preferably removed using a 20 to 100 µm cell strainer, and more preferably removed using a 20 to 40 µm cell strainer.

In this step (3), as in step (1), the temperature, pH, and dissolved oxygen concentration can be controlled to any desired value desirable for induction into NK cells by using a temperature sensor, a pH sensor, a dissolved oxygen sensor, and the like.

The histone methyltransferase inhibitor and/or AHR antagonist may be added to the medium in step (3) throughout step (3) or only for a part of the step. When the inhibitor (antagonist) is added only for a part of step (3), it can be added, for example, only in the first half of step (3) (e.g., until the time when IL-7 and Flt3L are removed from the medium). When the first half of step (3) is performed using a third medium containing IL-7 and Flt3L in addition to IL-15 and SCF, the inhibitor (antagonist) can be added to the medium until, for example, the time when IL-7 and Flt3L are removed from the medium, but not limited thereto.

As regards addition of a histone methyltransferase inhibitor and/or an AHR antagonist in step (3), no particular limitation is imposed on the selection of inhibitor (antagonist) as long as it can efficiently and rapidly induce NK cells and does not adversely affect the cells. In addition, a suitable concentration can be selected depending on the kind of inhibitor. The concentration of the histone methyltransferase inhibitor can be selected from the range of generally about 10 nM to about 5 µM, preferably about 100 nM to about 2 µM, more preferably about 500 nM to about 1 µM. The concentration of the AHR antagonist can be selected from the range of generally about 100 nM to about 10 µM, preferably about 500 nM to about 5 µM, more preferably about 1 µM to about 5 µM. Any one kind of the histone methyltransferase inhibitor may be used alone, or two or more kinds thereof may be used in combination. Any one kind of the AHR inhibitor may be used alone, or two or more kinds thereof may be used in combination.

In the method of the present invention, a step of expansion culture of the obtained NK cells or a step of maturation of NK cells may be further performed as step (4) after step (3). The expansion culture step or the maturation step can be performed by appropriately applying a known method.

The method of the present invention is characterized in that all of the above-mentioned steps (1) to (3) or (1) to (4) are performed without using a carrier for three-dimensional culture or an extracellular substrate.

The method of the present invention may further include a step of cryopreserving the obtained NK cells. After completion of the NK production step, the NK cells of the present invention can be frozen and preserved by a method known per se.

The present invention is described in more detail in the following with reference to Example. However, they are merely illustrative and the present invention is not limited thereto.

### [Example]

### Example 1: Induction of differentiation from pluripotent stem cells into NK cells

In this example, a human iPS cell line was used as the pluripotent stem cell, and the process from iPS cell sphere formation to NK cell differentiation was performed by a three-dimensional culture method. GSK126 or SR1 was added to the medium starting from the process of inducing pluripotent stem cell spheres into cell populations containing HPCs onward, and the results were compared with those without the addition. In the step using perfusion culture, a stirring blade was used to mix the culture medium, and a pH sensor, a temperature sensor, and a dissolved oxygen sensor were used as sensors, and various management items were continuously monitored and controlled while mixing the culture medium. In addition, the perfusion culture was managed by controlling the inflow and outflow rate per hour using a dedicated pump and a balance.

### step (1): Step of forming pluripotent stem cell spheres

For the formation of pluripotent stem cell spheres, iPS cells expanded in two dimensions were seeded into a three-dimensional culture container at a cell density of 0.25×10⁵ cells/mL. Other details are as in the following method:
The medium used in step (1) was StemFit (registered trademark) AK03N (Ajinomoto Healthy Supply Co., Ltd.) supplemented with the ROCK inhibitor Y27632 at 10 µM.

In the pluripotent stem cell sphere formation step, the culture container was a single-use bottle with a total volume of 500 mL, and the culture medium was continuously mixed using a stirring blade inside the container. The pH was continuously monitored with a pH sensor and controlled to 7.00-7.40 by supplying sterile medium, sterile air, or sterile carbon dioxide.

The dissolved oxygen concentration was continuously monitored using a dissolved oxygen sensor and controlled to maintain a concentration of not less than 1.50 mg/L by supplying sterile air or sterile oxygen gas. The culture temperature was continuously monitored using a temperature sensor and controlled to 37.0°C by heating the container from the outside with a heater. The culture medium volume was adjusted to 250 mL, and a fresh medium was supplied using a pump and a balance, and the culture medium was discharged at the same rate. The dilution rate was controlled at 0.04 hr⁻¹ from the third day to the fifth day of culture, and 0.06 hr⁻¹ from the fifth day to the tenth day of culture. The separation membrane used was made of SUS316 with a fine pore size of 25 µm.

On the tenth day of culture, the cell suspension containing spheres was collected from the culture medium, and the average particle size of the spheres and the sphere density were measured using a microscope.

### step (2): Step of inducing pluripotent stem cell spheres into cell population including hematopoietic progenitor cells

To produce hematopoietic progenitor cells, iPS cell spheres produced in step (1) were seeded in a three-dimensional culture container to a density of 30 spheres/mL. Other details were as in the following method.

As the medium, DMEM/F-12, HEPES (Thermo Fisher Scientific) supplemented with CHIR99021 at 2 µM, BMP4 at 80 ng/mL, VEGF165 at 80 ng/mL, bFGF at 50 ng/mL, and ROCK inhibitor Y27632 at 10 µM was used as base conditions (condition 1) until day 2, and this medium supplemented with SR1 at 2 µM (condition 2) or GSK126 at 1 µM (condition 3) was used as test conditions. From day 2 to day 4, Essential 6 (Thermo Fisher Scientific) supplemented with SCF at 50 ng/mL, VEGF165 at 80 ng/mL, SB431542 at 2 µM, bFGF at 50 ng/mL, and ROCK inhibitor Y27632 at 10 µM was used as base conditions (condition 1), and this medium supplemented with SR1 at 2 µM (condition 2) or GSK126 at 1 µM (condition 3) was used as test conditions. Furthermore, from day 4 to day 14, a general hematopoietic progenitor cell differentiation-inducing basal medium supplemented with SCF at 50 ng/mL and Flt3L at 50 ng/mL was used as base conditions (condition 1), and this medium supplemented with SR1 at 2 µM (condition 2) or GSK126 at 1 µM (condition 3) was used as test conditions.

In this step, the culture container was a single-use bottle with a total volume of 500 mL, and the culture medium was continuously mixed using a stirring blade inside the container. The pH was continuously monitored with a pH sensor and controlled to 7.07 by supplying sterile medium, sterile air, or sterile carbon dioxide.

The dissolved oxygen concentration was continuously monitored using a dissolved oxygen sensor and controlled to maintain a concentration of not less than 1.50 mg/L by supplying sterile air or sterile oxygen gas. The culture temperature was continuously monitored using a temperature sensor and controlled to 37.0°C by heating the container from the outside with a heater. The culture medium volume was adjusted to 250 mL, and a fresh medium was supplied using a pump and a balance, and the culture medium was discharged at the same rate. The dilution rate was controlled at 0.07 hr⁻¹ from the first day to the second day of culture, 0.06 hr⁻¹ from the second day to the fourth day of culture, 0.13 hr⁻¹ from the fifth day to the seventh day of culture, 0.07 hr⁻¹ from the seventh day to the twelfth day of culture, and 0.02 hr⁻¹ from the twelfth day to the fourteenth day of culture. The separation membrane used was made of SUS316 with a fine pore size of 75 µm.

On the 14th day of culture, the spheres were removed from the culture medium to obtain a suspension of floating cells, and the cell surface markers known to be expressed in hematopoietic progenitor cells (CD34, CD43, CD45, CD117) were confirmed by flow cytometry.

The production efficiency of hematopoietic progenitor cells was compared between the production with and without the addition of GSK126 and SR1, and no significant difference was found in the expression of the confirmed markers.

### step (3): Step of inducing hematopoietic progenitor cells into NK cells

NK cells were produced by seeding the culture medium containing hematopoietic progenitor cells produced in step (2) (culture medium on day 14 produced in step 2) into a three-dimensional culture container at an inoculation ratio of 25% (v/v), including spheres. Other details were as in the following method.

As the medium, AIM V Serum Free Medium (Thermo Fisher Scientific) with FBS added at a concentration of 5%, to which IL-15, IL-7, SCF, and Flt3L were added to a final concentration of 50 ng/mL, was used as base conditions (condition 1). The test conditions used were those in which SR1 was added at 2 µM thereto (condition 2) or GSK126 was added at 1 µM thereto (condition 3).

As the culture container, a 30 mL rotary stir container was always used, and suspension culture was performed at 55 rpm in a 5% CO₂ incubator at 37°C.

100 µL of the culture medium was collected during the culture, the density of the floating cells was measured using NC-202 (ChemoMetec), and the fold expansion from the fourth day of the start of culture was calculated (Fig. 1). Under condition 2 and condition 3 (respectively added with SR1 and GSK126), it was possible to culture the cells at a higher fold expansion after passaging than the control.

The NK cell culture produced by the method of the present invention was collected over time, and the expression of CD56 as a cell surface marker known to be expressed on NK cells, and the expression of CD14, known to be expressed on monocytes and macrophages, for confirmation of non-target cells, were confirmed by flow cytometry (Fig. 2 and Fig. 3). As a result, under condition 3 (with addition of GSK126), CD56 expression increased more rapidly than under condition 1, showing that NK cell can be produced more efficiently.

From Fig. 1, compared to base conditions, SR1 conferred higher cell proliferation efficiency in the early stages of the NK cell differentiation induction step. On the other hand, GSK126 conferred high cell proliferation efficiency in the later stages of the differentiation induction step, and the onset of differentiation into CD56-positive cells (NK cells or progenitor cells thereof) was earlier than under base conditions. Therefrom it is suggested that the combined use of SR1 and GSK126 will result in a synergistic or mutually complementing action of the effect of each drug alone, and a rapid onset of NK cell differentiation and increased proliferation efficiency throughout the entire differentiation induction step are expected.

### [Industrial Applicability]

The method for producing NK cells derived from pluripotent stem cell of the present invention can be said to be useful for the mass production of NK cells that can be used in cancer immunotherapy.

This application is based on a patent application No. 2023-126651 filed in Japan (filing date: August 2, 2023), the contents of which are incorporated in full herein.

## Claims

1. A method for producing a natural killer (NK) cell or a progenitor cell thereof from a pluripotent stem cell, comprising:
a) a step of inducing a hematopoietic progenitor cell (HPC) from a pluripotent stem cell in vitro;
b) a step of expanding HPC in vitro; and
c) a step of inducing an NK cell or a progenitor cell thereof from HPC in vitro, wherein
a histone methyltransferase and/or an aryl hydrocarbon receptor (AHR) are/is inhibited in one or more steps of steps a) to c).

2. The method according to claim 1, wherein the histone methyltransferase catalyzes the addition of a methyl group to histone 3 lysine residue 27 (H3K27).

3. The method according to claim 1, wherein the histone methyltransferase is EZH1 (enhancer of zeste homolog 1) and/or EZH2 (enhancer of zeste homolog 2).

4. The method according to claim 1, wherein the histone methyltransferase is inhibited by a small molecule inhibitor.

5. The method according to claim 4, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

6. The method according to claim 1, wherein the inhibition of AHR has antagonist activity against AHR.

7. The method according to claim 1, wherein the AHR is inhibited by a small molecule inhibitor.

8. The method according to claim 7, wherein the small molecule inhibitor is SR1 (StemRegenin 1), CH-223191, GNF-351, 7-ketocholesterol, CB7993113, 6,2',4'-trimethoxyflavone, PD98059, or BAY 2416964.

9. The method according to any one of claims 1 to 8, wherein one or more steps of the steps a) to c) are performed by a perfusion culture method.

10. A method for producing an NK cell or a progenitor cell thereof from a pluripotent stem cell, comprising
(1) a step of forming pluripotent stem cell spheres having an average particle size of not less than 200 µm in a first medium;
(2) a step of inducing the pluripotent stem cell spheres obtained in step (1) into a cell population containing HPCs by three-dimensional culture using a second medium containing a histone methyltransferase inhibitor and/or an AHR antagonist; and
(3) a step of inducing the cell population containing HPCs obtained in step (2) into a cell population containing NK cells or progenitor cells thereof by three-dimensional culture using a third medium containing a histone methyltransferase inhibitor and/or an AHR antagonist,
wherein one or more steps of steps (1) to (3) are performed by a perfusion culture method.

11. The method according to claim 10, wherein the histone methyltransferase catalyzes the addition of a methyl group to H3K27.

12. The method according to claim 10, wherein the histone methyltransferase is EZH1 and/or EZH2.

13. The method according to claim 10, wherein the histone methyltransferase inhibitor is a small molecule inhibitor.

14. The method according to claim 13, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

15. The method according to claim 10, wherein the AHR antagonist is a small molecule inhibitor.

16. The method according to claim 15, wherein the small molecule inhibitor is SR1, CH-223191, GNF-351, 7-ketocholesterol, CB7993113, 6,2',4'-trimethoxyflavone, PD98059, or BAY 2416964.

## Amended claims

### Amended claims under Art. 19.1 PCT

1. (amended) A method for producing a natural killer (NK) cell or a progenitor cell thereof from a pluripotent stem cell, comprising:
a) a step of inducing a hematopoietic progenitor cell (HPC) from a pluripotent stem cell in vitro;
b) a step of expanding HPC in vitro; and
c) a step of inducing an NK cell or a progenitor cell thereof from HPC in vitro, wherein
a histone methyltransferase and/or an aryl hydrocarbon receptor (AHR) are/is inhibited in one or more steps of steps a) to c), and one or more steps of steps a) to c) are performed by a perfusion culture method.

2. The method according to claim 1, wherein the histone methyltransferase catalyzes the addition of a methyl group to histone 3 lysine residue 27 (H3K27).

3. The method according to claim 1, wherein the histone methyltransferase is EZH1 (enhancer of zeste homolog 1) and/or EZH2 (enhancer of zeste homolog 2).

4. The method according to claim 1, wherein the histone methyltransferase is inhibited by a small molecule inhibitor.

5. The method according to claim 4, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

6. The method according to claim 1, wherein the inhibition of AHR has antagonist activity against AHR.

7. The method according to claim 1, wherein the AHR is inhibited by a small molecule inhibitor.

8. The method according to claim 7, wherein the small molecule inhibitor is SR1 (StemRegenin 1), CH-223191, GNF-351, 7-ketocholesterol, CB7993113, 6,2',4'-trimethoxyflavone, PD98059, or BAY 2416964.

9. (canceled)

10. A method for producing an NK cell or a progenitor cell thereof from a pluripotent stem cell, comprising
(1) a step of forming pluripotent stem cell spheres having an average particle size of not less than 200 µm in a first medium;
(2) a step of inducing the pluripotent stem cell spheres obtained in step (1) into a cell population containing HPCs by three-dimensional culture using a second medium containing a histone methyltransferase inhibitor and/or an AHR antagonist; and
(3) a step of inducing the cell population containing HPCs obtained in step (2) into a cell population containing NK cells or progenitor cells thereof by three-dimensional culture using a third medium containing a histone methyltransferase inhibitor and/or an AHR antagonist, wherein one or more steps of steps (1) to (3) are performed by a perfusion culture method.

11. The method according to claim 10, wherein the histone methyltransferase catalyzes the addition of a methyl group to H3K27.

12. The method according to claim 10, wherein the histone methyltransferase is EZH1 and/or EZH2.

13. The method according to claim 10, wherein the histone methyltransferase inhibitor is a small molecule inhibitor.

14. The method according to claim 13, wherein the small molecule inhibitor is GSK126, EPZ005687, GSK343, Tazemetostat (EPZ-6438), UNC1999, EBI-2511, PF-06726304, Lirametostat (CPI-1205), EPZ011989, CPI-169, CPI-360, GSK503, EI1, OR-SO, OR-S1, DS-3201, FT671, XL177A, P5091, HBX19818, Parthenolide, GNE-6640, XL188, or L55.

15. The method according to claim 10, wherein the AHR antagonist is a small molecule inhibitor.

16. The method according to claim 15, wherein the small molecule inhibitor is SR1, CH-223191, GNF-351, 7-ketocholesterol, CB7993113, 6,2',4'-trimethoxyflavone, PD98059, or BAY 2416964.
